(19) Europäisches Patentamt
European Patent Office
Office européen des brevets

(11) Veröffentlichungsnummer: **0 027 965**
**B1**

(12) # EUROPÄISCHE PATENTSCHRIFT

(45) Veröffentlichungstag der Patentschrift: **13.06.84**

(21) Anmeldenummer: **80106305.8**

(22) Anmeldetag: **16.10.80**

(51) Int. Cl.³: **C 07 C 127/19,**
C 07 D 237/14,
C 07 D 403/12,
C 07 D 265/30,
C 07 D 295/20,
C 07 D 207/06,
C 07 D 213/64,
A 01 N 47/30, A 01 N 47/38
//C07C93/14

(54) **Substituierte Harnstoffe, ihre Herstellung und Verwendung als Herbizide und Mittel dafür.**

(30) Priorität: **24.10.79 DE 2942930**

(43) Veröffentlichungstag der Anmeldung:
**06.05.81 Patentblatt 81/18**

(45) Bekanntmachung des Hinweises auf die Patenterteilung:
**13.06.84 Patentblatt 84/24**

(84) Benannte Vertragsstaaten:
**AT BE CH DE FR GB IT LI NL SE**

(56) Entgegenhaltungen:
GB - A - 921 682
GB - A - 1 423 376

CHEMICAL ABSTRACTS, Band 92, Nr. 13, 31. März 1980, Seite 214, Spalte 2, Nr. 105844f Columbus, Ohio,U.S.A.
CHEMICAL ABSTRACTS, Band 92, Nr. 21, 26. Mai 1980, Seite 175, Spalte 2, Nr. 175777k Columbus, Ohio, U.S.A.

(73) Patentinhaber: **BASF Aktiengesellschaft**
**Carl-Bosch-Strasse 38**
**D-6700 Ludwigshafen (DE)**

(72) Erfinder: **Parg, Adolf, Dr.**
**Paray-le-Monial-Strasse 8**
**D-6702 Bad Duerkheim (DE)**
Erfinder: **Wuerzer, Bruno, Dr. Dipl.-Landwirt**
**Ruedigerstrasse 13**
**D-6701 Otterstadt (DE)**
Erfinder: **Hamprecht, Gerhard, Dr.**
**Rote-Turm-Strasse 28**
**D-6940 Weinheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 027 965 B1

**Beschreibung**

Die vorliegende Erfindung betrifft neue substituierte Phenylharnstoffe, Verfahren zu deren Herstellung, Herbizide, welche diese Verbindungen enthalten, sowie Verfahren zur Bekämpfung unerwünschten Pflanzenwuchses mit diesen Verbindungen.

Es ist bereits bakannt, daß die herbizid wirksame Substanz N,N-Dimethyl-N'[4-(4-chlorphenoxy)-phenyl]-harnstoff eine gute Verträglichkeit für Möhren (Daucus carota) und Bohnen (Phaseolus vulgaris) besitzt (DE—A—11 42 251). Sie wird jedoch bislang nur in begrenztem Umfang bei Sojabohnen (Glycine max), Erdbeeren (Fragaria vesca) und bei einigen Zwiebelsorten (Allium cepa) oder auch bei Sellerie (Apium graveolens) angewendet (Herbicide Handbook of the Weed Science Society of America, 4th Ed., 1979). Dieses Herbizid besitzt jedoch den Nachteil, daß es als Nachauflaufmittel sehr früh ausgebracht werden muß und nur bei sehr jungen Entwicklungsstadien der Unkräuter befriedigend wirkt. Zud em kann seine Phytotoxizität für Sojabohnen und Zwiebeln stärkere und kritische Ausmaße annehmen.

Weitere substituierte 4-Phenoxyphenylharnstoffe mit selektiven herbiziden Eigenschaften wurden in der DE—A—19 01 501 beschrieben. Auch in der DE—A—24 11 320 sind herbizid wirksame (Trifluormethylphenoxy)-phenylharnstoffe beschrieben, welche jedoch lediglich für Möhren verträglich sind. Schließlich läßt der aus dem Wirkungsbeispiel der DE—A—25 38 178 bekannte und vorzugsweise im Nachauflaufverfahren einzusetzende N,N-Dimethyl-N'[4-(3-trifluormethylphenoxy)-phenyl]-harnstoff keinerlei Selektivität mehr erkennen.

Außerdem sind aus der GB—A—14 23 376 N-[3-(4-Trifluormethylphenoxy)-6-nitro-phenyl]-N-methyl-harnstoffe bekannt, die ebenfalls herbizid wirksam sind.

Es wurde gefunden, daß substituierte Harnstoffe der Formel I

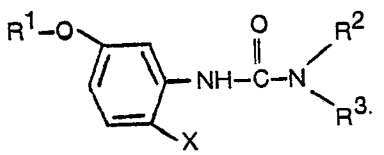

I

worin

$R^1$ entweder für einen substituierten Phenylrest der Formeln

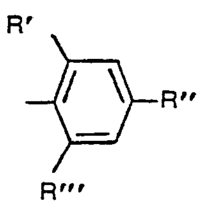

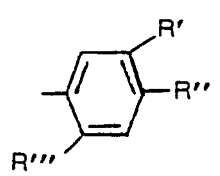 oder 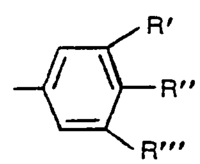 ,

in denen R', R'' und R''' jeweils unabhängig voneinander Wasserstoff, Halogen, Nitro, Niedrigalkyl, Halogenniedrigalkyl, Cyan, Niedrigalkoxy, Halogenniedrigalkoxy, Niedrigalkylmercapto, Niedrigalkylsulfinyl oder Niedrigalkylsulfonyl bedeuten oder für einen gegebenenfalls substituierten Heteroarylrest steht,

$R^2$ Wasserstoff, Niedrigalkyl, Niedrigalkoxy, Niedrigalkenyl, Niedrigalkinyl oder Cycloalkyl,

$R^3$ Wasserstoff oder Niedrigalkyl bedeuten, wobei $R^2$ und

$R^3$ zusammen auch für Oligomethylen oder Oxaoligomethylen stehen können, und

X Wasserstoff, Halogen, Nitro, Niedrigalkyl, Halogenniedrigalkyl, Niedrigalkoxy, Halogenniedrigalkoxy oder Cyan bedeuten, wobei jedoch X keine Nitrogruppe bedeutet, wenn $R^2$ und/oder $R^3$ Niedrigalkyl sind,

im Vergleich zu 4-phenoxysubstituierten Phenylharnstoffen neben einer beachtlichen herbiziden Aktivität noch eine überraschend gute Verträglichkeit gegenüber einer Reihe von Kulturpflanzen aufweisen.

Ist $R^1$ ein substituierter Phenylrest, so können R', R'' und R''' jeweils unabhängig voneinander bedeuten: Wasserstoff, Fluor, Chlor, Brom, Iod, Nitro, Cyan, Methyl, Ethyl, Trifluormethyl, Difluormethyl, Fluormethyl, Trichlormethyl, Dichlormethyl, Chlormethyl, Difluormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, Trichlormethoxy, Trifluormethoxy, 1-Chlorethoxy, 2-Chlorethoxy, 1-Fluorethoxy, 2-Fluorethoxy, 2,2,2-Trichlorethoxy, 2,2,2-Trifluorethoxy, 1,1,2,2-Tetrafluorethoxy, 1,1,2,2,2-Pentafluorethoxy, Methylmercapto, Ethylmercapto, Methylsulfinyl, Ethylsulfinyl, Methylsulfonyl oder Ethylsulfonyl. Unter diesen Resten sind Fluor, Chlor, Trifluormethyl und Trifluormethoxy bevorzugt.

Als Heteroarylreste sind für $R^1$ folgende bevorzugt:

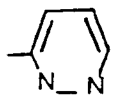 , 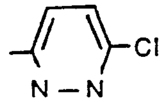 , 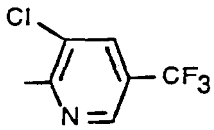 oder 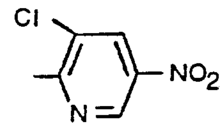

$R^2$ kann bedeuten: Wasserstoff, Methyl, Ethyl, Propyl, Isopropyl, tert.-Butyl, Methoxy, Ethoxy, Vinyl, Propen-1-yl, Allyl, Crotyl, Methallyl, Buten-1-yl-3, Buten-1-yl-4, Ethinyl, Propin-1-yl, Propargyl, Butin-2-yl-1, Butin-1-yl-3, Butin-1-yl-4, 3-Methyl-butin-1-yl-3, Cyclopropan, Cyclobutan, Cyclopentan oder Cyclohexan. Bevorzugt sind Methyl und Ethyl.

$R^3$ kann bedeuten: Wasserstoff und vorzugsweise Methyl oder Ethyl, Steht $R^2$ und $R^3$ für einen Oligomethylenrest, so kann $R^2$ und $R^3$ zusammen mit dem Stickstoff Pyrrolidinyl oder Piperidinyl bezeichnen.

X kann sein: Wasserstoff, Fluor, Chlor, Nitro, Methyl, Ethyl, Trichlormethyl, Trifluormethyl, Difluormethyl, Methoxy, Ethoxy, Trifluormethoxy, Trichlormethoxy oder Cyan, wobei jedoch X keine Nitrogruppe bedeutet, wenn $R^2$ und/oder $R^3$ Niedrigalkyl sind, Geeignet sind Wasserstoff und Nitro.

Weiterhin wurde gefunden, daß man die substituierten Phenylharnstoffe der Formel I erhält, wenn man

a) substituierte Aniline der Formel II

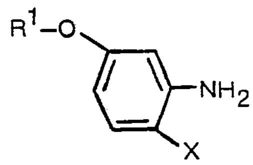 II,

in der $R^1$ und X die vorgenannten Bedeutungen besitzen, mit Phosgen und anschließend mit Aminen der Formel III

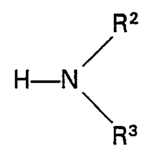 III,

in der $R^2$ und $R^3$ die vorgenannten Bedeutungen besitzen, umsetzt,

b) substituierte Aniline der Formel II mit einem Carbamidsäurechlorid der Formel IV

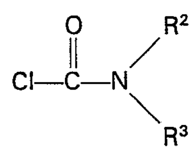 IV,

in der $R^2$ und $R^3$ die vorgenannten Bedeutungen besitzen, umsetzt,

c) substituierte Aniline der Formel II mit Alkylisocyanaten der Formel V

$$R^3—N=C=O \qquad V,$$

in der $R^3$ die vorgenannte Bedeutung außer Wasserstoff besitzt, umsetzt oder

d) 3-substituierte Benzoylhalogenide der Formel VI

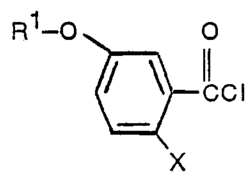 VI,

in welcher $R^1$ und X die vorgenannten Bedeutungen besitzen, mit einem Alkaliazid oder Trimethylsilylazid umsetzt und die so erhaltenen Säureazide in die entsprechenden Isocyanate überführt und letztere mit Aminen der Formel III umsetzt.

Alle vier Herstellungsverfahren a), b), c) und d) werden bevorzugt unter Mitverwendung geeigneter Lösungs- oder Verdünnungsmittel durchgeführt. Als solche kommen praktisch alle inerten

organischen Solventien in Frage. Hierzu gehören insbesondere: Kohlenwasserstoffe, wie Ligroin, Benzin, Toluol, Pentan, Hexan, Cyclohexan, Petrolether, Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o,m,p-Dichlorbenzol, o,m,p-Chlortoluol, Nitrokohlenwasserstoffe, wie Nitrobenzol, Nitroethan, o-,m-,p-Nitrotoluol, Nitrile, wie Acetonitril, Butyronitril, Isobutyronitril, Ether, wie Diethylether, Di-n-propylether, Tetrahydrofuran, Dioxan, Ester, wie Acetessigester, Ethylacetat, Isobutylacetat, ferner Ketone, wie Aceton, Methylethyl-, Methylisopropyl- und Methylisobutylketon, außerdem Amide, wie Methylformamid und Dimethylformamid.

Das Verfahren a) läßt sich sehr gut durchführen, indem man das substitiuerte Anilin der Formel II zunächst mit einem Überschuß Phosgen in das entsprechende Isocyanat (siehe Houben-Weyl, Band 8, 1952, Seite 122 bis 123 oder Methodicum Chimicum, Band 6, 1974, Seite 780) überführt und anschließend mit mindestens der äquivalenten Menge Amin der Formel III gegebenenfalls in Gegenwart eines Lösungsmittels bei einer Temperatur von —10°C bis 150°C, vorzugsweise von 20°C bis 120°C, kontinuierlich oder diskontinuierlich umsetzt.

Gemäß b) wird das substituierte Anilin der Formel II gemäß einer allgemeinen Vorschrift aus Houben-Weyl, Band 8, 1952, Seiten 160 bis 161 mit einem Überschuß von bis zu 20% N,N-Dialkylcarbamidsäurechlorid der Formel IV gegebenenfalls in Gegenwart eines Lösungsmittels und gegebenenfalls in Gegenwart eines Säurebinders bei einer Temperatur von —10° bis 150°C, vorzugsweise von 20° bis 120°C, kontinuierlich oder diskontinuierlich umgesetzt. Als Säurebinder können alle üblichen Säurebindemittel verwendet werden. Hierzu gehören vorzugsweise Alkalihydroxide, Alkalicarbonate, Alkalialkoholate und tertiäre organische Basen. Als besonders geeignet seien im einzelnen genannt: Natriumhydroxid, Natriumhydrogencarbonat, Natriummethylat, Triäthylamin, Pyridin, Trimethylamin, $\alpha,\beta\gamma$-Picolin, Lutidin, N,N-Dimethylanilin, N,N-Dimethylcyclohexylamin, Chinolin, Tri-n-propylamin, Tri-n-butylamin und Acridin.

Beim Verfahren c) wird das substituierte Anilin der Formel II mit einem Überschuß von bis zu 20% Isocyanat der Formel V, gegebenenfalls in Gegenwart eines Lösungsmittels bei einer Temperatur von —10° bis 150°C, vorzugsweise 20° bis 120°C, kontinuierlich oder diskontinuierlich umgesetzt (vgl. Houben-Weyl, Band 8, 1952, Seiten 157 bis 159).

Zur Durchführung des Verfahrens d) wird zunächst 0,1 Mol Benzoylchlorid mit 0,1 bis 0,4 Mol Alkaliazid oder Trimethylsilylazid im Überschuß zu dem entsprechenden Phenylisocyanat umgesetzt. Dieses wird gegebenenfalls durch Einengen des Reaktionsgemisches als Rohprodukt isoliert und für die weiteren Umsetzungen gegebenenfalls in Gegenwart eines Lösungsmittels mit der mindestens äquimolaren Menge Amin versetzt. Besonders gut geht das Verfahren, wenn man das Benzoesäurechlorid der Formel VI mit Trimethylsilylazid umsetzt, anschließend thermisch gemäß einer Vorschrift von H.R. Kricheldorf in Synthesis 1972, Seiten 551 bis 553 in das entsprechnede Isocyanat überführt und danach mit einem Amin der Formel III gegebenenfalls in Gegenwart eines Lösungsmittels bei einer Temperatur von —10°C bis 150°C, vorzugsweise von 20°C bis 120°C, kontinuierlich oder diskontinuierlich umsetzt.

Die Isolierung der Endprodukte erfolgt entweder bei aus dem Reaktionsgemisch ausgefallenen Stoffen durch Absaugen und anschließende Reinigung durch Umkristallisieren oder Chromatographieren oder durch Einengen des Reaktionsgemisches im Vakuum zur Trockne und Reinigung des Rückstandes durch Umkristallisieren oder Chromatographieren.

Die als Ausgangsstoffe für die Reaktionen a), b), c) verwendbaren substituierten Aniline der Formel II sind zum Teil neu. Sie können nach allgemein üblichen Methoden, die in der Literatur beschrieben sind, dargestellt werden.

Die für d) als Ausgangstoffe benötigten 3-substituierten Benzoesäurechloride der Formel VI können ebenfalls aus ihren bekannten Carbonsäuren (US-Patentschriften 4 031 131 und 3 652 645) bzw. nach den dort beschriebenen Methoden mit Hilfe chlorierender Agentien hergestellt werden.

Herstellung von Ausgangsstoffen:

a) 63,6 Teile 3-2'-Chlor-4'-trifluormethylphenoxy-1-nitrobenzol werden in 200 ml Ethanol gelöst, mit 10 g Raney-Nickel als Katalysator versetzt und bei 50°C und 100 atm Wasserstoff hydriert. Nach Abtrennen des Katalysators wird das Lösungsmittel im Vakuum abgezogen, der Rückstand in Ether aufgenommen, das Amin durch Begasen mit Salzsäure ausgefällt und isoliert. Durch Behandeln mit verdünnter Natronlauge kann das Amin freigesetzt werden. Man erhält 42,4 Teile (75% der Theorie) 3-(2'-Chlor-4'-trifluormethyl-phenoxy)-anilin mit $n_D^{25}$: 1,5542.

b) Zu einer Suspension von 35,2 Teilen Dikaliumsalz der 3-Hydroxybenzoesäure in 100 Teilen Dimethylsulfoxid werden bei 40°C 34,6 Teile 3,6-Dichlorpyridazin in 100 Teilen Dimethylsulfoxid portionsweise zugefügt. Anschließend rührt man 5 Stunden bei 90°C nach, kühlt ab, rührt die gesamte Reaktionsmischung in 1000 Teilen Wasser ein und säuert mit konzentrierter Salzsäure an. Der Niederschlag wird abgesaugt, und man erhält nach dem Trocknen 45 Teile (= 90% der Theorie) 3-(6'-Chlorpyridazinyl-3'-oxy)-benzoesäure mit dem Schmelzpunkt 187—192°C.

c) 26,7 Teile 3-(6'-Chlorpyridazinyl-3'-oxy)-benzoylchlorid werden in 100 Teilen absolutem Dioxan gelöst und mit 13,8 Teilen Trimethylsilylazid versetzt. Man erwärmt drei Stunden auf 90°C, kühlt ab, engt das Reaktionsgemisch unter Vakuum ein und erhält 24 Teile (= 100% der Theorie) 3-(6'-Chlorpyridazinyl-3'-oxy)-phenylisocyanat mit einem Schmelzpunkt von 90 95°C (Zers.) und einer

Reinheit von ca. 90%.

Die nach dieser Methode gewonnen Phenylisocyanate können ohne weitere Reinigung für die Darstellung der Harnstoffderivate eingesetzt werden.

Herstellung der Endprodukte:

Beispiel 1

14,4 Teile 3-2(2′-Chlor-4′-trifluormethyl-phenoxy)-anilin werden in 50 Teilen Acetonitril gelöst und bei 20°C mit 3 Tropfen Triethylamin und 3,2 Teilen Methylisocyanat ver setzt. Man rührt 2 Stunden bei Raumtemperatur nach, engt die Reaktionsmischung zur Trockne ein und kristallisiert den Rückstand aus Toluol um. Man erhält 10 Teile (=58% der Theorie) N-Methyl-N′-3-(2′-chlor-4′-trifluormethyl-phenoxy)-phenylharnstoff mit dem Schmelzpunkt 158—161°C.

Beispiel 2

Zu einer Lösung von 14,4 Teilen 3-(2′-Chlor-4′-trifluormethyl-phenoxy)-anilin in 25 Teilen Pyridin fügt man bei 10°C 7,5 Teile N,N-Dimethylcarbamidsäurechlorid portionsweise zu. Es wird eine halbe Stunde auf 70 bis 75°C erwärmt, das Pyridin zum größten Teil im Vakuum abgezogen, der Rückstand auf Wasser gegossen und mit verdünnter Salzsäure angesäuert. Der ölige Rückstand wird in Methylenchlorid aufgenommen, mit Magnesiumsulfat getrocknet und eingeengt. Nach der Chromatographie über Kieselgel mit Laufmittel Toluol/Aceton 70:30 erhält man 14 Teile (= 78% der Theorie) N,N-Dimethyl-N′-3-(2′-chlor-4′-trifluormethyl-phenoxy)-phenylharnstoff mit dem Schmelzpunkt 96—98°C.

Beispiel 3

12,4 Teile 3-(6′-Chlorpyridazinyl-3′-oxy)-phenylisocyanat werden in 150 ml Acetonitril gelöst und bei 20°C mit 3 Tropfen Triethylamin und 3,1 Teilen N-Methyl-O-methylhydroxylamin versetzt. Es wird 1 Stunde bei Raumtemperatur nachgerührt, die Reaktionsmischung unter Vakuum eingeengt und der Rückstand aus wenig Acetonitril umkristallisiert. Man erhält 12 Teile (= 79% der Theorie) N-Methyl-N-methoxy-N′-3-(6′-chlorpyridazinyl-3′-oxy)-phenylharnstoff mit einem Schmelzpunkt von 111—113°C.

Analog Beispiel 1—3 wurden die in der folgenden Übersicht angegebenen Verbindungen hergestellt:

| Beispiel Nr. | $R^1$ | $R^2$ | $R^3$ | X | Fp; $n_D^{25}$ |
|---|---|---|---|---|---|
| 4 | 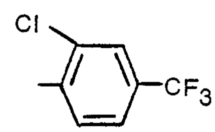 | —$C_2H_5$ | $C_2H_5$ | H | 1,5412 |
| 5 | " | —$CHCH_3$<br>C≡CH | $CH_3$ | H | 112—118°C |
| 6 | " | —$OCH_3$ | $CH_3$ | H | 1,5501 |
| 7 | " | —$CH_3$ | $C_3H_7$(i) | H | 132—134°C |
| 8 | 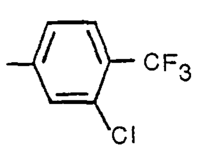 | $CH_3$ | $CH_3$ | H | 1,5524 |
| 9 | " | $C_2H_5$ | $C_2H_5$ | H | 1,5392 |
| 10 | " | $CH_3$ | H | H | 1,5502 |
| 11 | " | —$CHCH_3$<br>C≡CH | $CH_3$ | H | 1,5498 |

| Beispiel Nr. | $R^1$ | $R^2$ | $R^3$ | X | Fp; $n_D^{25}$ |
|---|---|---|---|---|---|
| 12 | 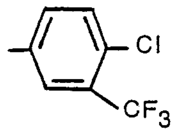 | $CH_3$ | $CH_3$ | H | 1,5601 |
| 13 | „ | $C_2H_5$ | $C_2H_5$ | H | 1,5513 |
| 14 | „ | $CH_3$ | H | H | 1,5637 |
| 15 | „ | $CHCH_3$<br>\|<br>$C\equiv CH$ | $CH_3$ | H | 1,5569 |
| 16 | 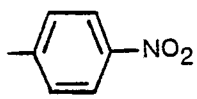 | $CH_3$ | $CH_3$ | H | |
| 17 | „ | $C_2H_5$ | $C_2H_5$ | H | 1,5920 |
| 18 | 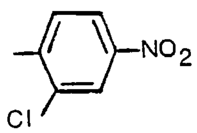 | $CH_3$ | $CH_3$ | „ | 164—166°C |
| 19 | „ | $C_2H_5$ | $C_2H_5$ | „ | |
| 20 | „ | $CHCH_3$<br>\|<br>$C\equiv CH$ | $CH_3$ | „ | $\nu_{c=o} = 1650\ cm^{-1}$ |
| 21 | „ | $CH_3$ | H | „ | |
| 22 | 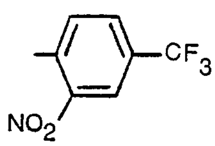 | „ | $CH_3$ | H | 135—139°C |
| 23 | „ | $C_2H_5$ | $C_2H_5$ | „ | 63—68°C |
| 24 | „ | $—CH_3$ | H | „ | |
| 25 | „ | $—CHCH_3$<br>\|<br>$C\equiv CH$ | $CH_3$ | „ | 1,5492 |
| 26 | 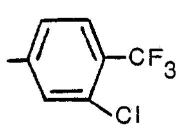 | $OCH_3$ | $CH_3$ | H | |
| 27 | 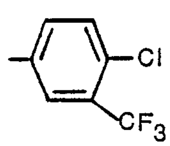 | „ | „ | H | |
| 28 | 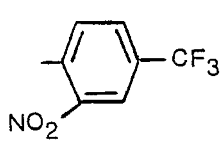 | „ | „ | „ | |

| Beispiel Nr. | $R^1$ | $R^2$ | $R^3$ | X | Fp; $n_D^{25}$ |
|---|---|---|---|---|---|
| 29 | 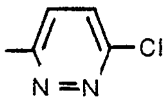 | $CH_3$ | $CH_3$ | H | 161—165°C |
| 30 | „ | „ | H | „ | 176—180°C |
| 31 | „ | $C_2H_5$ | $C_2H_5$ | „ | 113—117°C |
| 32 | „ | 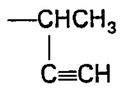 | $CH_3$ | „ | 164—165°C |
| 33 | 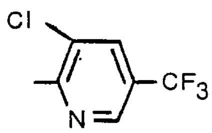 | $CH_3$ | $CH_3$ | H | |
| 34 | 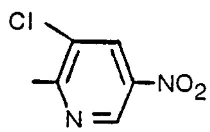 | „ | „ | „ | |
| 35 | 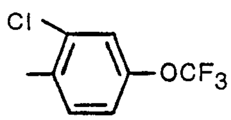 | $CH_3$ | $CH_3$ | „ | |
| 36 | „ | $C_2H_5$ | $C_2H_5$ | „ | |
| 37 | 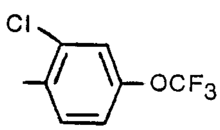 | $CH_3$ | H | H | |
| 38 | „ | 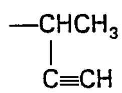 | $CH_3$ | „ | |
| 39 | 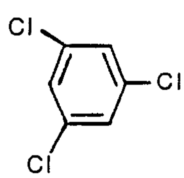 | $CH_3$ | „ | H | |
| 40 | 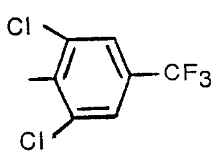 | $CH_3$ | $CH_3$ | H | |
| 41 | „ | $C_2H_5$ | $C_2H_5$ | H | |
| 42 | „ | $CH_3$ | $OCH_3$ | H | |

| Beispiel Nr. | $R^1$ | $R^2$ | $R^3$ | X | Fp; $n_D^{25}$ |
|---|---|---|---|---|---|
| 43 | 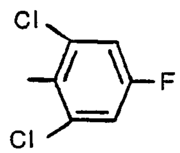 | $CH_3$ | $OCH_3$ | H | |
| 44 | 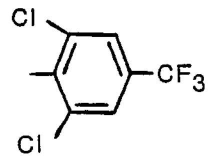 | „ | H | „ | |
| 45 | 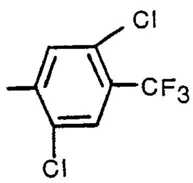 | „ | $CH_3$ | „ | |
| 46 | 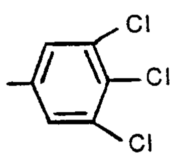 | $CH_3$ | $CH_3$ | H | |
| 47 | 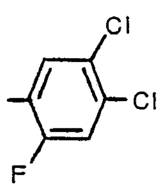 | „ | „ | „ | |
| 48 | 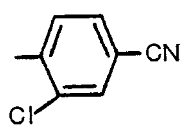 | „ | „ | „ | |
| 49 | 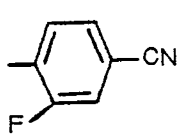 | „ | „ | H | |
| 50 | 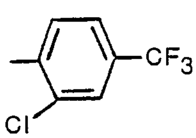 | H | $CH_2-CH(CH_3)_2$ | H | 1,5423 |
| 51 | „ | „ | $CH_3$<br>$-CH-C_2H_5$ | „ | 119—123°C |
| 52 | „ | $C_3H_7(i)$ | $C_3H_7(i)$ | „ | 118—122°C |

| Beispiel Nr. | $R^1$ | $R^2$ | $R^3$ | X | Fp; $n_D^{25}$ |
|---|---|---|---|---|---|
| 53 | 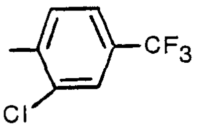 | | 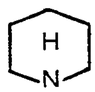 | H | 1,5619 |
| 54 | 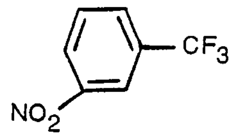 | | 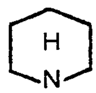 | „ | $\nu_{co} = 1640\ cm^{-1}$ |
| 55 | 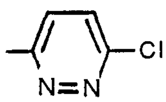 | H | $CH_3$ <br> $-CH-C_2H_5$ | „ | 180—182°C |
| 56 | 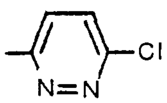 | $C_3H_7(i)$ | $C_3H_7(i)$ | H | 159—162°C |
| 57 | „ | $OCH_3$ | 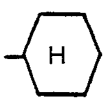 | „ | 148—152°C |
| 58 | „ | 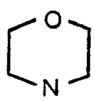 | | H | 194—196°C |

Die neuen Wirkstoffe besitzen insbesondere eine starke herbizide Aktivität und eine gute Verträglichkeit gegen Kulturpflanzen. Weiter weisen sie eine fungizide Wirksamkeit auf.

Ihre Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon und Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnapthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitblaugen und Methylcellulose.

Pulver, Streu- und Staubmittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kalk, Kreide, Talkum, Bolus, Löß, Ton, Dolomit, Diatomeenerde, Calcium- und Magnesiumsulfat, Magnesiumoxid und gemahlene Kunststoffe.

Beispiele für Formulierungen sind:

I 20 Gewichtsteile der Verbindung des Beispiels 1 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.

II 3 Gewichtsteile der Verbindung des Beispiels 2 werden mit 97 Gewichtsteilen feinteiligem Kaolin innig vermischt. Man erhält auf diese Weise ein Stäubemittel, das 3 Gew.% des Wirkstoffs enthält.

III 30 Gewichtsteile der Verbindung des Beispiels 3 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 4 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V 20 Teile der Verbindung des Beispiels 5 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-kondensates und 68 Teilen eines paraffinischen Mineralöls innig vermischt. Man erhält eine stabile ölige Dispersion.

VI Man vermischt 90 Gewichtsteile der Verbindung des Beispiels 1 mit 10 Gewichtsteilen N-Methyl-$\alpha$-pyrrolidon und erhält eine Lösung, die zur Anwendung in Form kleinster Tropfen geeignet ist.

VII 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzosulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII 20 Gewichtsteile der Verbindung des Beispiels 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX 20 Gewichtsteile der Verbindung des Beispiels 3 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Beispiele zur herbiziden Wirkung

Der Einfluß verschiedener Vertreter der erfindungsgemäßen Verbindungen auf das Wachstum von unerwünschten und von Kulturpflanzen wird in nachfolgenden Gewächshausversuchen demonstriert.

Als Kulturgefäße dienten Plastikblumentöpfe von 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorlaufanwendung das Aufbringen des Wirkstoffs auf die Erdoberfläche. Sie wurde hierbei in Wasser als Verteilungsmittel suspendiert oder emulgiert und mittels fein verteilender Düsen gespritzt. Nach dem Aufbringen der Mittel wurden die Gefäße leicht beregnet um Keimung un Wachstum in Gang zu bringen und auch gleichzeitig das chemische Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde.

TABELLE 1

Liste der Pflanzennamen

| Botanischer Name | Deutscher Name |
|---|---|
| Amaranthus retroflexus | Zurückgekrümmter Fuchsschwanz |
| Cassia spp. | Cassiaarten |
| Centaurea cyanus | Kornblume |
| Chenopodium album | Weißer Gänsefuß |
| Chrysanthemum segetum | Saatwucherblume |
| Cyperus ferax | |
| Echinochloa crus galli | Hühnerhirse |
| Euphorbia geniculata | Südamerik. Wolfsmilchart |
| Glycine max | Sojabohnen |
| Gossypium hirsutum | Baumwolle |
| Hordeum vulgare | Gerste |
| Lamium spp. | Taubnesselarten |
| Matricaria spp. | Kamillearten |
| Sesbania exaltata | Turibaum |
| Sinapis alba | Weißer Senf |
| Solanum nigrum | Schwarzer Nachtschatten |
| Sorghum bicolor | Mohrenhirse (Kulturhirse) |
| Triticum aestivum | Weizen |
| Zea mays | Mais |
| Chenopodium spp. | Gänsefußart |

Zum Zwecke der Nachlaufbehandlung zog man die Pflanzen je nach Wuchsform in den Versuchsgefäßen erst bis zu einer Höhe von 3 bis 10 cm an und behandelte sie dann. Eine Abdeckung unterblieb. Die Aufstellung der Versuche erfolgte im Gewächshaus, wobei für wärmeliebende Arten heißere Bereiche (25 bis 40°C) und für solche gemäßigter Klimate 15 bis 30°C bevorzugt wurden. Die Versuchsperiode erstreckte sich über 3 bis 6 Wochen. Während dieser Zeit wurden die Pflanzen gepflegt und ihre Reaktion auf die einzelnen Behandlungen wurde ausgewertet. Die folgenden Tabellen enthalten die Prüfsubstanzen, die jeweiligen Dosierungen in kg/ha aktiver Substanz (a.S.) und die Testpflanzenarten.

Bewertet wird nach einer Skala von 0 bis 100.

Dabei bedeutet 0 keine Schädigung oder normalen Auslauf und 100 kein Aufgang der Pflanzen bzw. völlige Zerstörung zumindest der oberirdischen Sproßteile.

TABELLE 2

Bekämpfung unerwünschter breitblättiger Pflanzen in Baumwolle bei Nachauflaufanwendung im Gewächshaus

| Testpflanzen | Wirkstoffe und Schädigung % Beispiel Nr. 10 0,25 kg/ha a.S. | Beispiel Nr. 8 0,25 kg/ha a.S. | Vergleichsmittel (N,N-Dimethyl-N'-[4-chlorphenoxy)-phenyl]-harnstoff 1,0 kg/ha a.S. |
|---|---|---|---|
| Gossypium hirsutum | 10 | 10 | 62 |
| Amaranthus retroflexus | 100 | 100 | 48 |
| Euphorbia geniculata | 95 | 95 | 32 |
| Sesbania exaltata | 80 | — | 35 |

0 = keine Schädigung
100 = Pflanzen abgestorben

TABELLE 3

Selektive Beseitigung breitblättiger Unkräuter in Soja und Mais bei Nachauflaufanwendung im Gewächshaus

Wirkstoffe und Schädigung % bei 1,0 kg/ha a.S.

| Testpflanzen | Beispiel Nr. 2 | Vergleichsmittel (N,N-Dimethyl-N'-[4-chlorphenoxy)-phenyl]-harnstoff |
|---|---|---|
| Glycine max | 15 | 32 |
| Zea mays | 13 | 10 |
| Cassia spp. | 100 | 0 |
| Chenopodium spp. | 99 | 52 |
| Sesbania exaltata | 80 | 35 |
| Sinapis alba | 90 | 99 |

0 = keine Schädigung
100 = Pflanzen abgestorben

TABELLE 4

Selektive Beseitigung unerwünschter Pflanzen in Getreide bei Nachauflaufanwendung im Gewächshaus

| Testpflanzen | Wirkstoffe, Aufwandmenge und Schädigung % Beispiel Nr. 6 0,5 kg/ha a.S. | Beispiel Nr. 30 0,25 kg/ha a.S. |
|---|---|---|
| Hordeum vulgare | 0 | 0 |
| Triticum aestivum | 0 | 0 |
| Amaranthus retroflexus | — | 80 |
| Chenopodium album | 98 | 98 |
| Lamium spp. | 80 | 90 |

0 = keine Schädigung 100 = Pflanzen abgestorben

TABELLE 5

Selektive Beseitigung von unerwünschten Pflanzen in verschiedenen Kulturen bei Vorauflaufanwendung im Gewächshaus

| Wirkstoff des Beispiels Nr. 31 Testpflanzen | Schädigung % bei 1,0 kg/ha a.S. |
|---|---|
| Hordeum vulgare | 0 |
| Glycine max | 9 |
| Gossypium hirsutum | 10 |
| Sorghum bicolor | 3 |
| Zea mays | 0 |
| Amaranthus retroflexus | 99 |
| Chrysanthemum segetum | 100 |
| Centaurea cyanus | 80 |
| Cyperus ferax | 100 |
| Echinochloa crus galli | 82 |
| Matricaria spp. | 95 |
| Solanum nigrum | 100 |

0 = keine Schädigung, 100 = Pflanzen abgestorben

TABELLE 6

Beispiel zur herbiziden Wirkung verschiedener neuer Verbindung bei Vorauflaufanwendung im Gewächshaus

| Wirkstoff des Beispiels Nr. | Testpflanzen und Schädigung % bei 3,0 kg/ha a.S. Sinapis alba |
|---|---|
| 3 | 100 |
| 4 | 100 |
| 8 | 100 |
| 9 | 100 |
| 10 | 100 |
| 12 | 100 |
| 29 | 100 |
| 30 | 100 |
| 32 | 100 |

Die Tabellen 2—6 präsentieren die selektive herbizide Wirkung der erfindungsgemäßen Verbindungen. Die Applikation kann im Vorauflaufverfahren oder mit besserem Erfolg bei Nachauflaufanwendungen erfolgen. Dabei können die Mittel auf den Standort aufgebracht werden bevor die unerwünschten Pflanzen aus den Samen gekeimt oder aus vegetativen Pflanzenteilen ausgetrieben haben oder sie werden auf die Blätter der unerwünschten Pflanzen und der Kulturpflanzen appliziert. Eine weitere Ausbringungstechnik besteht darin, daß die Wirkstoffe mit Hilfe der Spritzgeräte so gespritzt werden, daß die Blätter empfindlicher Kulturpflanzen nach Möglichkeit nicht getroffen werden während die Wirkstoffe auf die darunterliegende Bodenfläche oder dort wachsende unerwünschte Pflanzen gelangen (post-directed, lay-by).

Die Aufwandmengen betragen je nach Jahreszeit und Wachstumsstadium 0,1 bis 15 kg/ha und mehr, vorzugsweise zwischen 0,2 und 3 kg/ha, wobei sich die höheren Dosen zur totalen Bekämpfung von Pflanzenwuchs eignen.

In Anbetracht der Vielseitigkeit der Applikationsmethoden können die erfindungsgemäßen Mittel oder diese enthaltende Mischungen außer bei den in den Tabellen aufgeführten Nutzpflanzen noch in einer weiteren großen Zahl von Kulturen zur Beseitigung unerwünschten Pflanzenwuchses eingesetzt werden.

Im einzelnen seien folgende Nutzpflanzen gennant:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |

| Botanischer Name | Deutscher Name |
|---|---|
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapus var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |

| Botanischer Name | Deutscher Name |
|---|---|
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |

| Botanischer Name | Deutscher Name |
|---|---|
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Die neuen substituierten Harnstoffe können unter sich und mit zahlreichen Vertretern anderer herbizider oder wachstumsregulierender Wirkstoffgruppen gemischt und gemeinsam ausgebracht werden. Beispielsweise kommen als Mischungskomponenten Diazine, N-Phenyl-carbamate, Thiolcarbamate, Diurethane, Halogencarbonsäuren, Phenoxyfettsäuren, Triazine, Amide, Harnstoffe, Diphenylether, Triazone, Uracile, Benzofuranderivate und andere in Betracht. Solche Kombinationen dienen zur Verbreiterung des Wirkungsspektrums und erzielen zuweilen synergistische Effekte. Eine Reihe von Wirkstoffen, welche zusammen mit den neuen Verbindungen für verschiedenste Anwendungsbereiche sinnvolle Mischungen ergeben, werden beispielhaft aufgeführt.

5-Amino-4-chlor-2-phenyl-3(2*H*)-pyridazinon
5-Amino-4-brom-2-phenyl-3(2*H*)-pyridazinon
5-Amino-4-chlor-2-cyclohexyl-3(2*H*)-pyridazinon
5-Amino-4-brom-2-cyclohexyl-3(2*H*)-pyridazinon
5-Methylamino-4-chlor-2-(2-trifluormethylphenyl)-3(2*H*)-pyridazinon
5-Methylamino-4-chlor-2-(2-$\alpha,\alpha,\beta,\beta$-tetrafluorethoxyphenyl-3(2*H*)-pyridazinon
5-Dimethylamino-4-chlor-2-phenyl-3(2*H*)-pyridazinon
4,5-Dimethoxy-2-phenyl-3(2*H*)-pyridazinon
4,5-Dimethoxy-2-cyclohexyl-3(2*H*)-pyridazinon
4,5-Dimethoxy-2-(2-trifluormethylphenyl)-3(2*H*)-pyridazinon
5-Methoxy-4-chlor-2-(2-trifluormethylphenyl)-3(2*H*)-pyridazinon
5-Amino-4-brom-2-(2-methylphenyl)-3(2*H*)-pyridazinon
3-(1-Methyläthyl)-1H-2,1,3-benzothiadiazin-4(3*H*)-on-2,2-dioxid und Salze
3-(1-Methylethyl)-8-chlor-1H-2,1,3-benzothiadiazin-4(3*H*)-on-2,2-dioxid und Salze
3-(1-Methylethyl)-8-fluor-1H-2,1,3-benzothiadiazin-4(3*H*)-on-2,2-dioxid und Salze
3-(1-Methylethyl)-8-methyl-1H-2,1,3-benzothiadiazin-4(3*H*)-on-2,2-dioxid und Salze
1-Methoxymethyl-3-(1-methylethyl)-2,1,3-benzothiadiazin-4(3*H*)-on-2,2-dioxid
1-Methoxymethyl-8-chlor-3-(1-methylethyl)-2,1,3-benzothiadiazin-4(3*H*)-on-2,2-dioxid
1-Methoxymethyl-8-fluor-3-(1-methylethyl)-2,1,3-benzothiadiazin-4(3*H*)-on-2,2-dioxid
1-Cyan-8-chlor-3-(1-methylethyl)-2,1,3-benzothiadiazin-4(3*H*)-on-2,2-dioxid
1-Cyan-8-fluor-3-(1-methylethyl)-2,1,3-benzothiadiazin-4(3*H*)-on-2,2-dioxid

1-Cyan-8-methyl-3-(1-methylethyl)-2,1,3-benzothiadiazin-4(3*H*)-on-2,2-dioxid
1-Cyan-3-(1-methylethyl)-2,1,3-benzothiadiazin-4(3*H*)-on-2,2-dioxid
1-Azidomethyl-3-(1-methylethyl)-2,1,3-benzothiadiazin-4(3*H*)-on-2,2-dioxid
3-(1-methyläthyl)-1H-(pyridino-[3,2-e]2,1,3-thiadiazin-(4)-on-2,2-dioxid
N-(1-Ethylpropyl)-2,6-dinitro-3,4-dimethylanilin
N-(1-Methylethyl)-N-ethyl-2,6-dinitro-4-trifluormethylanilin
N-n-Propyl-N-$\beta$-chlorethyl-2,6-dinitro-4-trifluormethylanilin
N-n-Propyl-N-cyclopropylmethyl-2,6-dinitro-4-trifluormethyl-anilin
N-Bis(n-propyl)-2,6-dinitro-3-amino-4-trifluormethylanilin
N-Bis(n-propyl)-2,6-dinitro-4-methyl-anilin
N-Bis(n-propyl)-2,6-dinitro-4-methylsulfonyl-anilin
N-Bis(n-propyl)-2,6-dinitro-4-aminosulfonyl-anilin
Bis($\beta$-chlorethyl)-2,6-dinitro-4-methyl-anilin
N-Ethyl-N-(2-methylallyl)-2,6-dinitro-4-trifluormethylanilin
N-Methylcarbaminsäure-3,4-dichlorbenzylester
N-Methylcarbaminsäure-2,6-di(tert.butyl)-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid
Ethyl-N-(3-(N'-phenylcarbamoyloxy)-phenyl)-carbamat
Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbamat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-N'-3-chlor-4-fluorphenylcarbamoyloxy)-phenyl)-carbamat
Ethyl-N-(3-N'-3,4difluorphenylcarbamoyloxy)-phenyl)-carbamat
Methyl-N-(3-(N'-3,4difluorphenylcarbamoyloxy)-phenyl)-carbamat
N-3-(4-Fluorphenoxycarbonylamino)-phenylcarbaminsäure-methylester
N-3-(2-Methylphenoxycarbonylamino)-phenylcarbaminsäure-ethylester
N-3-(4-Fluorphenoxycarbonylamino)-phenylthiolcarbaminsäure-methylester
N-3-(2,4,5-Trimethylphenoxycarbonylamino)-phenylthiolcarbaminsäure-methylester
N-3-(Phenoxycarbonylamino)-phenylthiolcarbaminsäure-methylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolylmethylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,1,1]-heptyl-thiolcarbaminsäure-ethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydrol-1-H-azepin-1-carbothiolat
S-Benzyl-3methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chloroallylester
N-Methyl-dithiocarbaminsäure-Natrium
Trichloressigsäure-Na-salz
$\alpha,\alpha$-Dichlorpropionsäure-Na-salz
$\alpha,\alpha$-Dichlorbuttersäure-Na-salz
$\alpha,\alpha,\beta,\beta$-Tetrafluorpropionsäure-Na-salz
$\alpha$-Methyl,$\alpha,\beta$-dichlorpropionsäure-Na-salz

α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephtalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4′-Chlorphenoxy)-phenoxy]-propionsäureisobutylester
2-[4-(2′,4′-Dichlorphenoxy)-phenoxy]-propionsäuremethylester
2-[4-(4′-Trifluormethylphenoxy)-phenoxy]-propionsäuremethylester
2-[4-(2′-Chlor-4′-trifluorphenoxy)-phenoxy]-propionsäure-Na-salz
2-[4-(3′,5′-Dichlorpyridyl-2-oxy)-phenoxy]-propionsäure-Na-salz
2-(N-Benzoyl-3,4-dichlorphenylamino)-propionsäureethylester
2-(N-Benzoyl-3-chlor-4-fluorphenylamino)-propionsäuremethylester
2-(N-Benzoyl-3-chlor-4-fluorphenylamino)-propionsäureisopropylester
2-Chlor-4-ethylamino-6-isopropylamino-1,3,5-triazin
2-Chlor-4-ethylamino-6-(amino-2′-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.-butyl-3-methylthio-4,5-dihydro-1,2,4-triazin-5-on
1-Methyl-3-cyclohexyl-6-dimethylamino-1,3,5-triazin-2,4-dion
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil
2-Methyl-4-(3′-trifluormethylphenyl)-tetrahydro-1,2,4-oxadiazin-3,5-dion
2-Methyl-4-(4′-fluorphenyl)-tetrahydro-1,2,4-oxadiazin-3,5-dion
3-Amino-1,2,4-triazol
1-Allyloxy-1-(4-bromphenyl)-2-[1′,2′,4′-triazolyl-(1′)-]-ethan (Salze)
[-1-(1,2,4-Triazolyl-1′)]-[1(4′-chlorphenoxy)]-3,3-dimethylbutan-2-on
N,N-Diallylchloracetamid
N-Isopropyl-2-chloracetanilid
N-(Butin-1-yl-3)-2-chloracetanilid
2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazon-1-yl-methyl)-2-chloracetanilid
2,6-Dimethyl-N-(4-methylpyrazol-1-yl-methyl)-2-chloracetatanilid
2,6-Dimethyl-N-(1,2,4-triazol-1-yl-methyl)-2-chloracetanilid

2,6-Dimethyl-N-(3,5-dimethylpyrazol-1-yl-methyl)-2-chloracetanilid
2,6-Dimethyl-N-(1,3-dioxalan-2-yl-methyl)-2-chloracetanilid
2,6-Dimethyl-N-(2-methoxyethyl)-2-chloracetanilid
2,6-Dimethyl-N-(isobutoxymethyl)-2-chloroacetanilid
2,6-Diethyl-N-(methoxymethyl)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(propoxyethyl)-2-chloracetanilid
2-(2-Methyl-4-chlorphenoxy-N-methoxy-acetamid
2-($\alpha$-Naphtoxy)-N,N-diethylpropionamid
2,2-Diphenyl-N,N-dimethylacetamid
$\alpha$-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
N-2,4-Dimethyl-5-(trifluormethyl)-sulfonylamino-phenylacetamid
N-4-Methyl-5-(trifluormethyl)-sulfonylamino-phenylacetamid
2-Propionyl-amino-4-methyl-5-chlor-thiazol
O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-l-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Na-Salz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenyl-ether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$0,$^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernsteinsäureimid
2-Methyl-4,6-dinitrophenol (Salze, Ester)
2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-($\alpha$,$\alpha$-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-chlorphenyl)-1-benzoyl-3,3-dimethyl-harnstoff

1-(4-chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-$\alpha,\alpha,\beta$-$\beta$-Tetrafluoräthoxyphenyl)-3,3-dimethyl-harnstoff
1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4(4'-methoxyphenoxy)-phenyl]-3,3-dimethyl-harnstoff
1-Cyclooktyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-iso-butylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4methylphenylsulfonyl)-oxy]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
$\alpha$-Naphthoxyessigsäuremethylester.
2-(2-Methylphenoxy)propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)buttersäure (Salze, Ester, Amide)
Cyclohexyl-3-(2,4-dichlorphenoxy-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)

Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
0,0-Diisopropyl-5-(2-benzosulfonylamino-ethyl)-phosphordithionat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid
2-Chlor-4-trifluormethyl-3-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid
2-Chlor-4-trifluormethylphenyl-3'-2-fluorethoxy-4'-nitrophenylether
2-Chlor-4-trifluormethylphenyl-3(ethoxycarbonyl)methylthio-4-nitrophenylether
2,4,6-Trichlorphenyl-3(ethoxycarbonyl)methylthio-4-nitrophenylether
2-[1-(N-Ethoxamino)-butyliden]-5-(2-ethylthiopropyl)-3-hydroxy-cyclohexen-(2)-on-(1) (Salze)
2-[1-(N-Ethoxamino)-butyliden]-5-(2-phenylthiopropyl)-3-hydroxy-cyclohexen-(2)-on-(1) (Salze)

Außerdem ist es nützlich, die neuen erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse sind ferner die Mischbarkeit mit Mineralstofflösungen, welche zur Behebung von Ernährungs- oder Spurenelementmängeln eingesetzt werden. Zu den Einzelwirkstoffen oder Mischungen können auch Öle verschiedenen Typs, Ölkonzentrate, Netz- oder Haftmittel sowie Antischaummittel zugesetzt werden.

Als Fungizide wirken die neuen Verbindungen insbesondere gegen Rostkrankheiten; so ist beispielsweise eine protektive Sproßbehandlung gegen Erysiche graminis an Gerste und Weizen möglich.

## Patentansprüche

1. Substituierte Harnstoffe der Formel I

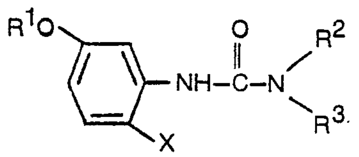
I,

worin

$R^1$ entweder für einen substituierten Phenylrest der Formeln

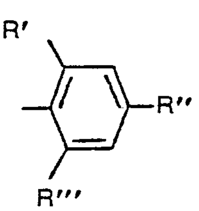
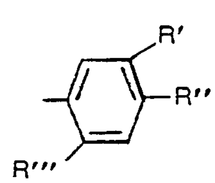
oder
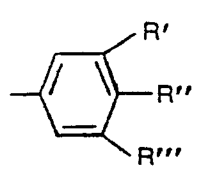
,

in denen R', R'' und R''' jeweils unabhängig voneinander Wasserstoff, Halogen, Nitro, Niedrigalkyl, Halogenniedrigalkyl, Cyan, Niedrigalkoxy, Halogenniedrigalkoxy, Niedrigalkylmercapto, Niedrigalkylsulfinyl oder Niedrigalkylsulfonyl bedeuten oder für einen gegebenenfalls substituierten Heteroarylrest steht,

$R^2$ Wasserstoff, Niedrigalkyl, Niedrigalkoxy, Niedrigalkenyl, Niedrigalkinyl oder Cycloalkyl,
$R^3$ Wasserstoff oder Niedrigalkyl bedeuten, wobei $R^2$ und
$R^3$ zusammen auch für Oligomethylen oder Oxaoligomethylen stehen können, und
X Wasserstoff, Halogen, Nitro, Niedrigalkyl, Halogenniedrigalkyl, Niedrigalkoxy, Halogenniedrigalkoxy oder Cyan bedeutet, wobei jedoch X keine Nitrogruppe bedeutet, wenn $R^2$ und/oder $R^3$ Niedrigalkyl sind.

2. Verbindung, ausgewählt aus der Gruppe, bestehend aus
N,N-Dimethyl-N'-3-(2-chlor-4-trifluormethylphenoxy)-phenylharnstoff,
N,N-Diethyl-N'-3-(2-chlor-4-trifluormethylphenoxy)-phenylharnstoff,
N-Methyl-N-methoxy-N'-3-(2-chlor-4-trifluormethyl-phenoxy)-phenylharnstoff,
N-Methyl-N-isobutinyl-N'-3-(2-chlor-4-trifluormethylphenoxy)-phenylharnstoff,
N-Methyl-N-isopropyl-N'-3-(2-chlor-4-trifluormethylphenoxy)-phenylharnstoff,
N-Methyl-N'-3-(2-chlor-4-trifluormethylphenoxy)-phenylharnstoff,
N,N-Dimethyl-N'-3-(3-chlor-4-trifluormethylphenoxy)-phenylharnstoff,
N,N-Diethyl-N'-3-(3-chlor-4-trifluormethylphenoxy)-phenylharnstoff,
N-Methyl-N-isobutinyl-N'-3-(3-chlor-4-trifluormethylphenoxy)-phenylharnstoff,
N,N-Dimethyl-N'-3-(3-trifluormethyl-4-chlorphenoxy)-phenylharnstoff,
N,N-Diethyl-N'-3-(3-trifluormethyl-4-chlorphenoxy)-phenylharnstoff,
N-Methyl-N-isobutinyl-N'-3-(3-trifluormethyl-4-chlorphenoxy)-phenylharnstoff,
N,N-Dimethyl-N'-3-(2-nitro-4-trifluormethylphenoxy)-phenylharnstoff,
N,N-Diethyl-N'-3-(2-nitro-4trifluormethylphenoxy)-phenylharnstoff,
N-Methyl-N-isobutinyl-3-(2-nitro-4-trifluormethylphenoxy)-phenylharnstoff,
N,N-Dimethyl-N'-3-(2-chlor-4-nitrophenoxy)-phenylharnstoff,
N,N-Diethyl-N'-3-(2-chlor-4-nitrophenoxy)-phenylharnstoff,
N-Methyl-N-isobutinyl-N'-3-(2-chlor-4-nitrophenoxy)-phenylharnstoff,
N,N-Dimethyl-N'-3-(6-chlor-pyridazinyl-3-oxy)-phenylharnstoff,
N,N-Diethyl-N'3-(6-chlor-pyridazinyl-3-oxy)-phenylharnstoff und
N-Methyl-N-methoxy-N'-3-(6-chlor-pyridazinyl-3-oxy)-phenylharnstoff.

3. Herbizid enthaltend mindestens einen substituierten Harnstoff der Formel I gemäß Anspruch 1.

4. Herbizid enthaltend mindestens einen substituierten Harnstoff der Formel I gemäß Anspruch 1 sowie einen festen oder flüssigen Trägerstoff.

5. Verfahren zur Herstellung von substituierten Harnstoffen der Formel I gemäß Anspruch 1, dadurch gekennzeichnet, daß man

a) substituierte Aniline der Formel II

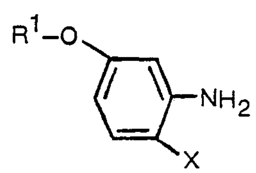

II,

in der $R^1$ und X die vorgenannten Bedeutungen besitzen, mit Phosgen und anschließend mit Aminen der Formel III

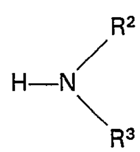

III,

in der $R^2$ und $R^3$ die vorgenannte Bedeutungen besitzen, umsetzt,

b) substituierte Aniline der Formel II mit einem Carbamidsäurechlorid der Formel IV

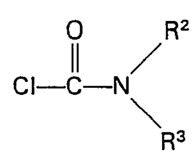

IV,

in der $R^2$ und $R^3$ die vorgenannten Bedeutungen besitzen, umsetzt,

c) substituierte Aniline der Formel II mit Alkylisocyanaten der Formel V

$$R^3—N{=}C{=}O \qquad V,$$

in denen $R^3$ die vorgenannte Bedeutung außer Wasserstoff besitzt, umsetzt oder

d) 3-substituierte Benzoylhalogenide der Formel VI

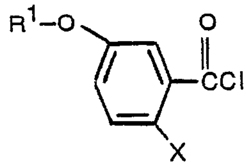

VI,

in welcher $R^1$ und X die vorgenannten Bedeutungen besitzen, mit einem Alkaliazid oder Trimethylsilylazid umsetzt und die so erhaltenen Säureazide in die entsprechenden Isocyanate überführt und letztere mit Aminen der Formel III umsetzt.

6. Verfahren zur Bekämpfung von unerwünschtem Pflanzenwachstum, dadurch gekennzeichnet, daß man die unerwünschten Pflanzen oder durch unerwünschtes Pflanzenwachstum bedrohte Flächen mit einer herbizid wirksamen Menge einer Verbindung der Formel I gemäß Anspruch 1 behandelt.

**Revendications**

1. Urées substituées de formule I

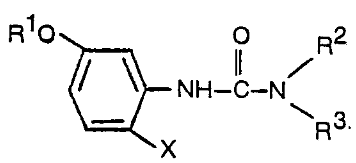

I,

où $R^1$ représente, soit un reste phényle substitué de formule

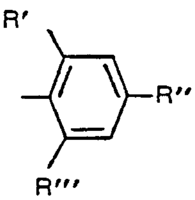

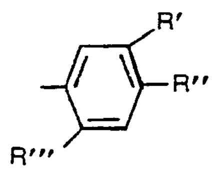

ou

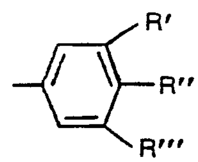

dans lesquelles R′, R″ et R‴ représentent, chaque fois, indépendamment les uns des autres, hydrogène, halogène, nitro, alkyle inférieur, halogénalkyle inférieur, cyano, alcoxy inférieur, halogénalcoxy inférieur, mercaptoalkyle inférieur, sulfinylalkyle inférieur ou sulfonylalkyle inférieur, soit un reste hétéroaryle éventuellement substitué,

$R^2$ représente hydrogène, alkyle inférieur, alcoxy inférieur, alcényle inférieur, alkinyle inférieur ou cycloalkyle,

$R^3$ représente hydrogène ou alkyle inférieur,

$R^2$ et $R^3$ pouvant aussi représenter ensemble oligométhylène ou oxaoligométhylène et

X représente hydrogène, halogène, nitro, alkyle inférieur, halogènealkyle inférieur, alcoxy inférieur, halogènealcoxy inférieur ou cyano, X ne représentant toutefois pas de groupe nitro, lorsque $R^2$ et/ou $R^3$ sont alkyle inférieur.

2. Composé choisi dans le groupe constitué par:
N,N-diméthyl-N′-3-(2-chloro 4 trifluorométhylphénoxy)-phénylurée,
N,N-diéthyl-N′-3-(2-chloro-4-trifluorométhylphénoxy)-phénylurée,
N-méthyl-N-méthoxy-N′-3-(2-chloro-4-trifluorométhylphénoxy)-phénylurée,
N-méthyl-N-isobutinyl-N′-3-(2-chloro-4-trifluorométhylphénoxy)-phénylurée,
N-méthyl-N-isopropyl-N′-3-(2-chloro-4-trifluorométhylphénoxy)-phénylurée,
N-méthyl-N′-3-(2-chloro-4-trifluorométhylphénoxy)-phénylurée,
N,N-diméthyl-N′-3-(3-chloro-4-trifluorométhylphénoxy)-phénylurée,
N,N-diéthyl-N′-3-(3-chloro-4-trifluorométhylphénoxy)-phénylurée,
N-méthyl-N-isobutinyl-N′-3-(3-chloro-4-trifluorométhylphénoxy)-phénylurée,
N,N-diméthyl-N′-3-(3-trifluorométhyl-4-chlorophénoxy)-phénylurée,
N,N-diéthyl-N′-3-(3-trifluorométhyl-4-chlorophénoxy)-phénylurée,
N-méthyl-N-isobutinyl-N′-3-(3-trifluorométhyl-4-chlorophénoxy)phénylurée,
N,N-diméthyl-N′-3-(2-nitro-4-trifluorométhylphénoxy)-phénylurée,
N,N-diéthyl-N′-3-(2-nitro-4-trifluorométhylphénoxy)-phénylurée,
N-méthyl-N-isobutinyl-3-(2-nitro-4-trifluorométhylphénoxy)-phénylurée,
N,N-diméthyl-N′-3-(2-chloro-4-nitrophénoxy)-phénylurée,
N,N-diéthyl-N′-3-(2-chloro-4-nitrophénoxy)phénylurée,

N-méthyl-N-isobutinyl-N'-3-(2-chloro-4-nitrophénoxy)-phénylurée,
N,N-diméthyl-N'-3-(6-chloro-pyridazinyl-3-oxy)-phénylurée,
N,N-diéthyl-N'-3-(6-chloro-pyridazinyl-3-oxy)-phénylurée et
N-méthyl-N-méthoxy-N'-3-(6-chloro-pyridazinyl-3-oxy)-phénylurée.

3. Herbicide contenant au moins une urée substituée de formule I selon la revendication 1.

4. Herbicide contenant au moins une urée substituée de formule I selon la revendication 1 ainsi qu'un véhicule solide ou liquide.

5. Procédé de préparation d'urées substituées de formule I selon la revendication 1, caractérisé par le fait qu'on fait réagir

a) des anilines substituées de formule II

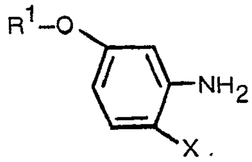

II,

dans laquelle $R^1$ et X ont les significations sus-indiquées, avec du phosgène et ensuite avec des amines de formule III

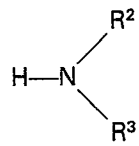

III

dans laquelle $R^2$ et $R^3$ ont les significations sus-indiquées.

b) des anilines substituées de formule II avec un chlorure d'acide carbamidique de formule IV

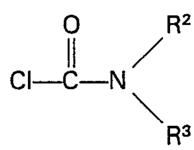

IV

dans laquelle $R^2$ et $R^3$ ont les significations sus-indiquées.

c) des anilines substituées de formule II avec des alkylisocyanates de formule V

$$R^3—N=C=O \qquad V$$

dans laquelle $R^3$ à la signification sus-indiquée, sauf hydrogène, ou

d) des halogénures de benzoyle substituées en 3, de formule VI

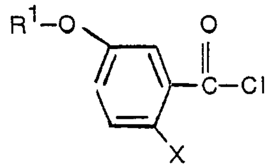

VI

dans laquelle $R^1$ et X ont les significations sus-indiquées, avec un azide alcalin ou triméthylsilylazide et l'on transforme les azides d'acide ainsi obtenus en les isocyanates correspondants, et on fait réagir ces derniers avec des amines de formule III.

6. Procédé pour lutter contre la croissance de plantes indésirables, caractérisé par le fait qu'on traite les plantes indésirables ou les surfaces menacées par la croissance de plantes indésirables avec une quantité, efficace au point de vue herbicide, d'un composé de formule I selon la revendication 1.

**Claims**

1. A substituted urea of the formula I

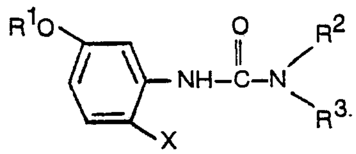

I,

where $R^1$ denotes substituted phenyl of one of the formulae

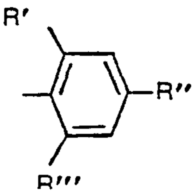

,

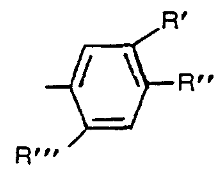

or

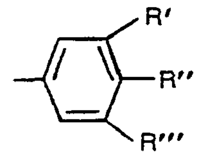

,

R′, R″ and R‴ being identical or different and each denoting hydrogen, halogen, nitro, lower alkyl, halo-lower alkyl, cyano, lower alkoxy, halo-lower alkoxy, lower alkylmercapto, lower alkylsulfinyl or lower alkylsulfonyl, or $R^1$ denotes substituted or unsubstituted heteroaryl, $R^2$ denotes hydrogen, lower alkyl, lower alkoxy, lower alkenyl, lower alkynyl or cycloalkyl, $R^3$ denotes hydrogen or lower alkyl, $R^2$ and $R^3$ together also being able to denote oligomethylene or oxaoligomethylene, and X denotes hydrogen, halogen, nitro, lower alkyl, halo-lower alkyl, lower alkoxy, halo-lower alkoxy, or cyano, but does not denote nitro if $R^2$ and/or $R^3$ are lower alkyl.

2. A compound selected from the group consisting of
N,N-dimethyl-N′-3-(2-chloro-4-trifluoromethylphenoxy)-phenylurea,
N,N-diethyl-N′-3-(2-chloro-4-trifluoromethylphenoxy)-phenylurea,
N-methyl-N-methoxy-N′-3-(2-chloro-4-trifluoromethylphenoxy)-phenylurea,
N-methyl-N-isobutynyl-N′-3-(2-chloro-4-trifluoromethylphenoxy)-phenylurea,
N-methyl-N-isopropyl-N′-3-(2-chloro-4-trifluoromethylphenoxy)-phenylurea,
N-methyl-N′-3-(2-chloro-4-trifluoromethylphenoxy)-phenylurea,
N,N-dimethyl-N′-3-(3-chloro-4-trifluoromethylphenoxy)phenylurea,
N,N-diethyl-N′-3-(3-chloro-4-trifluoromethylphenoxy)-phenylurea,
N-methyl-N-isobutynyl-N′-3-(3-chloro-4-trifluoromethylphenoxy)-phenylurea,
N,N-dimethyl-N′-3-(3-trifluoromethyl-4-chlorophenoxy)-phenylurea,
N,N-diethyl-N′-3-3(3-trifluoromethyl-4-chlorophenoxy)-phenylurea,
N-methyl-N-isobutynyl-N′-3-(3-trifluoromethyl-4-chlorophenoxy)phenylurea,
N,N-dimethyl-N′-3-(2-nitro-4-trifluoromethylphenoxy)-phenylurea,
N,N-diethyl-N′-3-(2-nitro-4-trifluoromethylphenoxy)-phenylurea,
N-methyl-N-isobutynyl-3-(2-nitro-4-trifluoromethylphenoxy)-phenylurea,
N,N-dimethyl-N′-3-(2-chloro-4-nitrophenoxy)-phenylurea,
N,N-diethyl-N′-3-(2-chloro-4-nitrophenoxy)-phenylurea,
N-methyl-N-isobutynyl-N′-3-(2-chloro-4-nitrophenoxy)-phenylurea,
N,N-dimethyl-N′-3-(6-chloropyridazinyl-3-oxy)-phenylurea,
N,N-diethyl-N′-3-(6-chloropyridazinyl-3-oxy)-phenylurea, and
N-methyl-N-methoxy-N′-3-(6-chloropyridazinyl-3-oxy)-phenylurea.

3. A herbicide containing at least one substituted urea of the formula I as claimed in claim 1.

4. A herbicide containing at least one substituted urea of the formula I as claimed in claim 1 and a solid or liquid carrier.

5. A process for the manufacture of a substituted urea of the formula I as claimed in claim 1, wherein

a) a substituted aniline of the formula II

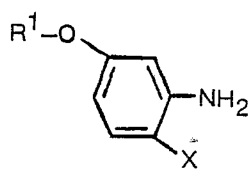

II,

where $R^1$ and X have the above meanings, is reacted with phosgene and then with an amine of the formula III

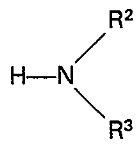

III,

where $R^2$ and $R^3$ have the above meanings;

b) a substituted aniline of the formula II is reacted with a carbamic acid chloride of the formula IV

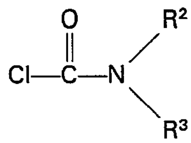

IV,

where $R^2$ and $R^3$ have the above meanings;

c) a substituted aniline of the formula II is reacted with an alkyl isocyanate of the formula V

$$R^3—N=C=O \qquad V,$$

where $R^3$ has, with the exception of hydrogen, the above meaning; or

d) a 3-substituted benzoyl halide of the formula VI

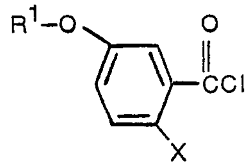

VI,

where $R^1$ and X have the above meanings, is reacted with an alkali metal azide or trimethylsilyl azide, the acid azide thus obtained is converted into the corresponding isocyanate, which is then reacted with an amine of the formula III.

6. A process for combating unwanted plant growth, wherein the unwanted plants, or areas threatened by unwanted plant growth, are treated with a herbicidally effective amount of a compound of the formula I as claimed in claim 1.